(19) **Europäisches Patentamt**

**European Patent Office**

**Office européen des brevets**

(11) **EP 1 303 758 B1**

(12) **EUROPEAN PATENT SPECIFICATION**

(45) Date of publication and mention
of the grant of the patent:
**27.12.2006 Bulletin 2006/52**

(51) Int Cl.:
*G01N 33/487* (2006.01)     *G01N 27/403* (2006.01)
*G01N 27/413* (2006.01)     *G01N 27/49* (2006.01)

(21) Application number: **01952446.1**

(22) Date of filing: **06.07.2001**

(86) International application number:
**PCT/US2001/021314**

(87) International publication number:
**WO 2002/006788 (24.01.2002 Gazette 2002/04)**

(54) **ELECTROCHEMICAL METHOD FOR MEASURING CHEMICAL REACTION RATES**

ELEKTROCHEMISCHES VERFAHREN ZUR MESSUNG CHEMISCHER REAKTIONSRATEN

PROCEDE ELECTROCHIMIQUE POUR MESURER DES VITESSES DE REACTIONS CHIMIQUES

(84) Designated Contracting States:
**AT BE CH CY DE DK ES FI FR GB GR IE IT LI LU MC NL PT SE TR**

(30) Priority: **14.07.2000 US 615691**
**14.07.2000 US 616433**
**14.07.2000 US 616512**
**14.07.2000 US 616556**

(43) Date of publication of application:
**23.04.2003 Bulletin 2003/17**

(73) Proprietor: **LifeScan, Inc.**
**Milpitas, CA 95035-6312 (US)**

(72) Inventors:
• **HODGES, Alastair**
**Blackburn South**
**Victoria 3130 (AU)**
• **CHATELIER, Ron**
**San Diego, CA 92126 (US)**

(74) Representative: **Mercer, Christopher Paul et al**
**Carpmaels & Ransford**
**43, Bloomsbury Square**
**London WC1A 2RA (GB)**

(56) References cited:
**WO-A-97/00441**          **WO-A-97/18465**
**WO-A-99/46585**          **US-A- 4 711 245**
**US-A- 5 141 868**        **US-A- 5 863 400**

## Description

Field of the Invention

The present invention relates to the measurement of the progress of a chemical reaction that generates an electroactive reaction product that is subsequently detected at an electrode amperometrically or coulometrically. The method is useful in applications where it is desirable to follow the progress of a chemical reaction, particularly in sensor applications where the progress of the reaction of an analyte can be useful in determining the analyte concentration.

Background of the Invention

Description of the Related Art

In amperometric electrochemistry the current flowing at the electrode can be used as a measure of the concentration of electroactive species being reacted electrochemically at the working electrode. In coulometry the current flowing at the electrode is integrated over time to give a total amount of charge passed which yields a measure of the amount of electroactive material reacted at the working electrode. The current flowing (or charge passed at any time) at the electrode is dependent upon the rate of transfer of the electroactive species to the working electrode. When a significant concentration of electroactive species is situated close to the electrode and an electrical potential is applied to the electrode sufficient to electrochemically react the electroactive species at the electrode/solution interface, initially a higher current will flow which will diminish with time. For an isolated electrode, where the potential applied to the electrode is sufficient to react the electroactive species effectively instantaneously upon arriving at the electrode and the transfer of electroactive species to the electrode is controlled by diffusion, the current will follow a curve known in the art as the Cottrell Equation. According to this equation the current varies inversely with the square root of time. This yields a current which decays with time as the electroactive species that reacts at the electrode becomes depleted close to the electrode and so electroactive species has to travel from further and further away to reach the electrode as time progresses.

If in addition to the electrochemical reaction of the electroactive species at the electrode the electroactive species is being generated close to the working electrode by a chemical reaction, the form of the current flowing at the electrode becomes complex. The electrode reaction tends to decrease the concentration of electroactive species close to the working electrode whereas the chemical reaction tends to increase the concentration of the electroactive species in this region. The time dependent behavior of these two processes therefore mix and it is difficult to measure the chemical reaction kinetics from the current flowing (or charge passed) at the electrode.

For this reason, in the published literature, the rates of chemical reactions are not generally measured electrochemically except in specialized applications using specialized equipment. An example of such equipment is known in the art as a rotating ring/disc electrode. This apparatus is only applicable to relatively fast reaction kinetics and requires that the electrode be rotated at a known controlled rate with well-characterized liquid hydrodynamics.

WO 97/18465 discloses a method for determining the concentration of a reduced or oxidized form of a redox species in an electrochemical cell comprising a working electrode and a counter electrode spaced from the working electrode such that reaction products from the counter electrode arrive at the working electrode.

US 5,863,400 discloses an electrochemical cell and a method of detecting and measuring an analyte using such a device

WO 97/00441 discloses an apparatus and a method for determining the concentration of a reduced (or oxidised) form of a redox species in an electrochemical cell comprising a working electrode and a counter electrode spaced from the working electrode by a predetermined distance.

WO 99/46585 discloses a method for determining the concentration of an analyte in a sample comprising the steps of heating the sample and measuring the concentration of the analyte or the concentration of a species representative thereof in the sample at a predetermined point on a reaction profile by means that are substantially independent of temperature.

US 5,141,868 discloses capillary fill cell devices to facilitate electrically-monitored tests, for example conductivity measurements, specific ion analysis, enzymatic reactions, and specific binding assays, using very small liquid samples.

US 4,711,245 discloses equipment and methods for detecting the presence of, measuring the amount of, and/or monitoring the level of, one or more selected components in a liquid mixture, employing an electrode sensing system.

Summary of the Invention

The method provided allows for the extraction of chemical reaction rate information using a simple electrochemical method, as defined in claim 1.

In a preferred embodiment, the method is used for measuring a rate of a chemical reaction between glucose and PQQ dependent glucose dehydrogenase in whole blood including providing an electrochemical cell having a working electrode, a counter electrode, at least one wall, a redox mediator including ferricyanide and contained within the electrochemical cell, and a reagent including PQQ dependent glucose dehydrogenase, the reagent being substantially immobilized in the electrochemical cell at a site at a minimum distance from the working electrode; placing the whole blood sample in the electrochemical cell such that the sample is in contact with the reagent, the redox mediator, the working electrode, and the counter electrode; reacting the glucose with the PQQ dependent glucose dehydrogenase to produce reduced PQQ dependent glucose dehydrogenase, the reduced PQQ dependent glucose dehydrogenase in turn reacting with the ferricyanide redox mediator to form ferrocyanide; applying a potential between the working electrode and the counter electrode, wherein the potential is sufficient to electrochemically react the ferrocyanide at the working electrode; and measuring the current produced by the electrochemical reaction of ferrocyanide at the working electrode, wherein the measurement is indicative of the rate of the chemical reaction between glucose and PQQ dependent glucose dehydrogenase.

Brief Description of the Drawings

Figure 1 depicts an electrochemical cell wherein the reagent is situated on a wall of the cell facing the working electrode.
Figure 2 depicts an electrochemical cell wherein the reagent is situated on the counter electrode.
Figure 3 shows current as a function of time for three whole blood samples for a reaction system including glucose, PQQ dependent glucose dehydrogenase and potassium ferricyanide redox mediator. The three blood samples contain haematocrit levels of 20%, 42%, and 65%, respectively, where the haematocrit is the volume percent of red blood cells in the sample.

Detailed Description of the Preferred Embodiment

The following description and examples illustrate a preferred embodiment of the present invention in detail. Those of skill in the art will recognize that there are numerous variations and modifications of this invention that are encompassed by its scope. Accordingly, the description of a preferred embodiment should not be deemed to limit the scope of the present invention.

According to the present invention, information relating to the rate of a chemical reaction that yields at least one electro active product can be obtained using an electrochemical cell by ensuring that the chemical reaction is localized at a site remote from the electrode used to electrochemically react the electroactive product (s).

Methods and devices for obtaining electrochemical measurements of fluid samples are discussed further in US 6,638,415 entitled "ANTIOXIDANT SENSOR", and US 6,632,349, entitled "HEMOGLOBIN SENSOR."

The site of the chemical reaction is sufficiently removed from the electrode such that the mass transfer of the electroactive species from the chemical reaction site to the electrode effectively controls the current flowing at the electrode at any time. This arrangement ensures a substantially linear electroactive species concentration gradient between the chemical reaction site and the electrode. The concentration of the electroactive species is maintained at effectively zero at the electrode by the electrochemical reaction taking place there. The time course of the magnitude of this concentration gradient will therefore be substantially determined only by the time course of the concentration of the electroactive specie (s) at the chemical reaction site and the diffusion coefficient (s) of the electroactive reaction product (s) in the liquid medium. Since the current flowing at the electrode is proportional to the concentration gradient of the electroactive specie(s) at the electrode, the time course of this current will reflect the time course of the chemical reaction occurring at the remote site. This allows the current measured at the electrode (or charge passed if the current is integrated) to be a used as a convenient measure of the rate and extent of the chemical reaction taking place.

An example of a suitable method for ensuring that the chemical reaction is remote from the working electrode is to immobilize one or more of the reaction components on a solid surface remote from the electrode. The reaction component(s) can be immobilized by incorporating them in a polymeric matrix that is dried on or otherwise attached to the solid surface. The reaction component(s) can also be tethered directly to the solid surface either by chemical or physical bonding. Alternatively one or more of the reaction components can simply be dried onto the solid surface without special immobilization means. In this situation one or more of the reaction components is sufficiently low in mobility, in the liquid matrix filling the electrochemical cell, that it does not migrate substantially from the position where it was dried during the time period that the electrochemical current can be usefully monitored to perform the required measurement. In this context substantial migration means that the slowest moving component required for the chemical reaction approaches closely enough to the working electrode that Cottrell type depletion kinetics begin to effect the time course of the current flowing at the electrode.

The range of separation distance between the chemical reaction site and the working electrode in preferred embodiments is desirably less than about 1 cm, preferably less than 5 mm, more preferably between 5, 10, 50, 100, 200, 500 microns and 5 mm, more preferably between 5, 10, 50, 100, 200 and 500 microns, and most preferably between 5, 10, 50, 100 and 200 microns.

As well as the working electrode, at least a counter electrode in contact with the liquid sample is provided to complete the electrochemical circuit. Optionally the counter electrode can function as a combined counter/reference electrode or a separate reference electrode can be provided. In a preferred embodiment, the working electrode and counter electrode are desirably spaced apart at a distance greater than about 300 microns, preferably at a distance greater than about 500 microns, more preferably at a distance between about 500 microns and 10 mm, more preferably at a distance between about 500 microns and 1, 2, 5 mm, and most preferably between 1 mm and 2, 5,10 mm.

The working electrode is constructed of materials that do not react chemically with any component with which it will come into contact during use to an extent that interferes with the current response of the electrode. If the working electrode is to be used as an anode then examples of suitable materials are platinum, palladium, carbon, carbon in combination with inert binders, iridium, indium oxide, tin oxide, mixtures of indium and tin oxide. If the working electrode is to be used as a cathode then in addition to the material listed above other suitable materials are steel, stainless steel, copper, nickel, silver and chromium.

Examples of materials suitable for the counter electrode are platinum, palladium, carbon, carbon in combination with inert binders, iridium, indium oxide, tin oxide, mixture of indium and tin oxide, steel, stainless steel, copper, nickel, chromium, silver and silver coated with a substantially insoluble silver salt such as silver chloride, silver bromide, silver iodide, silver ferrocyanide, silver ferricyanide.

The site of the chemical reaction can be localized on a bare wall or on the counter electrode, remote from the working electrode. The site of the chemical reaction can be on the same plane as the working electrode or more preferably in a plane facing and substantially parallel to the working electrode.

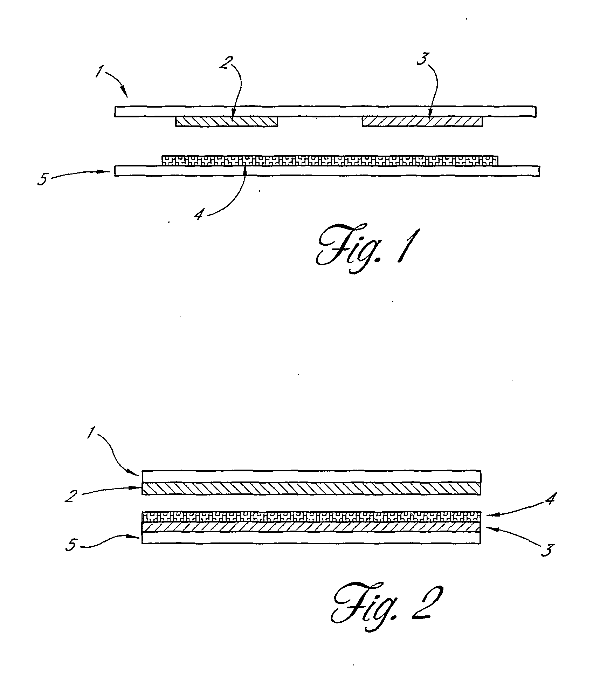

Figure 1 depicts an apparatus suitable for use with one embodiment. In Figure 1, a working electrode 2 and a counter electrode 3 are disposed on an electrically insulating substrate 1. On a second substrate 5 is disposed a layer of chemical reactants 4, where at least one of the reactants is substantially immobilized on the substrate 5. In use, the space between walls 1 and 5 is filled with a liquid containing a substance which is capable of reacting with the reagents 4 to produce at least one electroactive species. The products of the chemical reaction diffuse towards the working electrode 2 where the electroactive specie(s) are electrochemically reacted to produce a current. The magnitude of the current or the charge passed at a particular time, or the time course of the current or charge passed can then be used to obtain a measure of the rate or extent of the chemical reaction occurring at the reactant layer 4.

Figure 2 depicts another embodiment. The numbering of the components in Figure 2 correspond to the components in Figure 1. In Figure 2 the reactants 4 are disposed on the counter electrode 3 which is disposed on an electrically resistive substrate 5. In this embodiment the materials of construction of the counter electrode 3 are inert to reaction with any of the components of the reactants 4 disposed on the electrode 3.

An example of a chemistry and reaction that is suitable for use in a preferred embodiment is measuring glucose in whole blood using the enzyme PQQ dependent glucose dehydrogenase (GDHpqq) and a redox mediator. In this reaction glucose in the blood reacts with GDHpqq to form gluconic acid. In the process, the PQQ in the enzyme is reduced. A mediator, such as potassium ferricyanide, then oxidizes the PQQ in the enzyme and forms ferrocyanide. The enzyme in the oxidized form can then react with further glucose. The net effect of this reaction is to produce two ferrocyanide molecules for each glucose molecule reacted. Ferrocyanide is an electroactive species, and so can be oxidized at an electrode to produce a current. Other suitable enzymes for this reaction are glucose oxidase (G0D) or NAD dependent glucose dehydrogenase. For other reactions, lactate dehydrogenase and alcohol dehydrogenase may be used. Other suitable redox mediators include ferrocinium, osmium complexes with bipyridine, and benzophenone.

The reaction of glucose in whole blood with the enzyme can be slow, taking up to a few minutes to go to completion. Also, the higher the haematocrit of the blood sample, the slower the reaction. The haematocrit of the blood is the volume fraction of red cells in the whole blood sample. In this example, an electrochemical cell according to Figure 2 was constructed. A solution containing 50 mg/ml GDHpqq, 0.9 M potassium ferricyanide and 50 mM buffer at pH 6.5 was deposited on the counter electrode and the water removed to leave a dried reactant layer. In this layer the GDHpqq is large enough to be effectively immobilized on the counter electrode, whereas the ferricyanide can mix more evenly throughout the liquid in the electrochemical cell. The blood sample was introduced into the cell and a potential of +300 mV immediately applied between the working electrode and the counter electrode. Although a potential of +300 mV is most preferred for oxidizing ferrocyanide, the potential is desirably between +40 mV and +600 mV, preferably between +50 mV and +500 mV, and more preferably between +200 mV and +400 mV. In the cell, the working electrode consisted of a layer of gold sputtered onto a polyester substrate and the counter electrode consisted of a layer of palladium sputtered onto a polyester substrate.

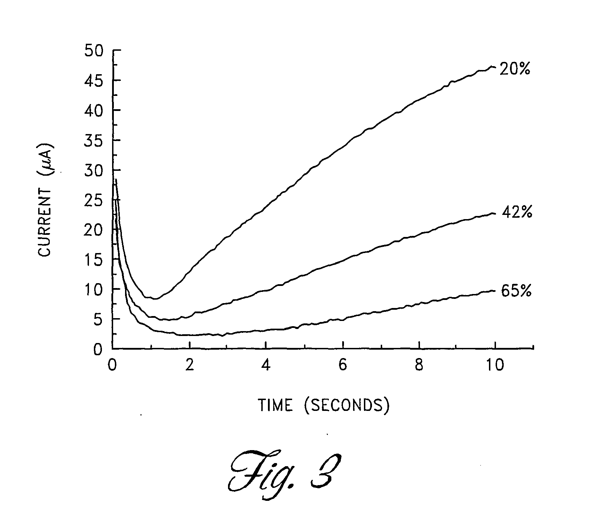

The current traces recorded for blood samples of different haematocrits, showing a faster rate of reaction in lower haematocrit blood, are given in Figure 3. The number at the end of each line is the percent haematocrit of the blood sample used, i.e., 20%, 42%, and 65%, respectively. The glucose level in each blood sample is approximately the same, namely 5.4 mM for the 65% haematocrit sample, 5.5 mM for the 42% haematocrit sample, and 6.0 mM for the 20% haematocrit sample.

The current shown in Figure 3 can be approximately given by the equation:

$$i = -FADC/L$$

where i is the current, F is Faraday's constant (96486.7 C/mole), A is the electrode area, D is the diffusion coefficient of the ferrocyanide in the sample, C is the concentration of ferrocyanide at the reaction site and L is the distance between the reaction site and the electrode. The reaction rate, given by the rate of change of C with time is therefore given by:

$$dC/dt = -(L/FAD)di/dt.$$

For the reactions depicted in Figure 3, between 6 and 8 seconds for the 20%, 42% and 65% haematocrit samples, the average di/dt was 3.82, 2.14 and 1.32 microamps/second, respectively. The diffusion coefficients of ferrocyanide for these samples were $2.0 \times 10^6$, $1.7 \times 10^6$ and $1.4 \times 10^6$ cm$^2$/sec for 20%, 42%, and 65% haematocrit samples, respectively. The electrode area was 0.1238 cm$^2$ and L was 125 microns. These values yield reaction rates of 2.0, 1.3, and 0.99 mM/second for the 20%, 42%, and 65% haematocrit samples, respectively.

The method described above may be used with any suitable electrochemical system. For example, the method may be used for measuring oxidants or antioxidants in fluid samples. The method is applicable to any oxidant or antioxidant that exists in a usefully representative concentration in a fluid sample. Antioxidants that may be analyzed include, for example, sulfur dioxide and ascorbic acid. Oxidants that may be analyzed include, for example, chlorine, bromine iodine, peroxides, hypochlorite, and ozone. Water insoluble oxidants or antioxidants may also be analyzed if an aqueous form can be prepared, e.g., by using a detergent to prepare an emulsion of the water insoluble redox reactive analyte. The method may also be applied to antibody/antigen reactions or to hemoglobin analysis.

The above description discloses several methods and materials of the present invention. This invention is susceptible to modifications in the methods and materials, as well as alterations in the fabrication methods and equipment. Such modifications will become apparent to those skilled in the art from a consideration of this disclosure or practice of the invention disclosed herein. Consequently, it is not intended that this invention be limited to the specific embodiments disclosed herein, but that it cover all modifications and alternatives coming within the true scope of the invention as embodied in the attached claims.

## Claims

1. A method for measuring a rate of a chemical reaction between a component of a liquid sample and a reagent, the reaction producing an electroactive species, comprising:

   providing an electrochemical cell having a working electrode (2), a counter electrode (3) and at least one wall (1);
   substantially immobilizing the reagent (4) in the electrochemical cell at a site (5) at a minimum distance from the working electrode (2), wherein the distance is such that transfer of the electroactive species from the site (5) to the working electrode (2) is diffusion controlled;
   placing the liquid sample in the electrochemical cell such that the liquid sample is in contact with the reagent (4), the working electrode (2) and the counter electrode (3);
   reacting the component with the reagent (4) to produce the electroactive species;
   applying a potential between the working electrode (2) and the counter electrode (3), wherein the potential is sufficient to electrochemically react the electroactive species at the working electrode (2); and
   measuring the current produced by the electrochemical reaction at the working electrode (2) to obtain a measure of the rate of the chemical reaction,

   wherein the rate of the chemical reaction is determined in accordance with the equation dC/Dt = (L/FAD)di/dt, where i is the current, F is Faraday's constant, A is the electrode area, D is the diffusion coefficient of the electroactive species in the sample, C is the concentration of electroactive species at the site and L is the distance between the site and the electrode.

2. The method according to claim 1, wherein the working electrode (2) and the counter electrode (3) are sufficiently spaced such that a product of an electrochemical reaction occurring at the counter electrode (3) does not reach the working electrode (2) while the current is measured.

3. The method according to claim 2, wherein the working electrode (2) and the counter electrode (3) are spaced apart at a distance greater than about 500 microns, preferably between 500 microns and 5 mm, more preferably between 1 mm and 2 mm.

4. The method according to claim 2 or claim 3, wherein the working electrode (2) and the counter electrode (3) are situated on the same plane.

5. The method according to any one of claims 1 to 4, wherein the site (5) and the working electrode (2) are separated by a minimum distance ranging from 10 microns to 5 millimeters, preferably from 50 microns to 500 microns, more preferably from 100 microns to 200 microns.

**6.** The method of any one of claims 1 to 5, wherein the counter electrode (3) is capable of functioning as a combined counter reference electrode.

**7.** The method of any one of claims 1 to 5, wherein the electrochemical cell further comprises a reference electrode.

**8.** The method according to any one of claims 1 to 7, wherein the working electrode (2) functions as an anode.

**9.** The method according to claim 8, wherein the working electrode (2) comprises platinum, palladium, carbon, carbon in combination with one or more inert binders, iridium, indium oxide, tin oxide, indium in combination with tin oxide or a mixture thereof.

**10.** The method according to any one of claims 1 to 7, wherein the working electrode (2) functions as a cathode.

**11.** The method according to any one of claims 1 to 10, wherein the counter electrode (3) comprises silver coated with a substantially insoluble silver salt.

**12.** The method according to claim 11, wherein the silver salt is silver chloride, silver bromide, silver iodide, silver ferrocyanide, or silver ferricyanide.

**13.** The method according to any one of claims 1 to 12, wherein the site (5) is situated on the counter electrode (3).

**14.** The method according to any one of claims 1 to 13, wherein the site (5) is situated on the wall.

**15.** The method according to any one of claims 1 to 14, wherein the site (5) and the working electrode (2) are situated on the same plane.

**16.** The method according to any one of claims 1 to 14, wherein the site (5) is situated in a plane facing and substantially parallel to the working electrode (2).

**17.** The method according to any one of claims 1 to 16, wherein the reagent (4) is contained within a polymeric matrix attached to a surface in the electrochemical cell.

**18.** The method according to any one of claims 1 to 16, wherein the reagent (4) is chemically tethered or physically tethered to a surface in the electrochemical cell.

**19.** The method according to any one of claims 1 to 16, wherein the reagent (4) is dried onto a surface in the electrochemical cell, the reagent (4) exhibiting sufficiently low mobility in the liquid sample such that the reagent (4) does not substantially migrate while the current is measured.

**20.** The method according to any one of claims 1 to 19, wherein the reagent (4) further comprises a redox mediator.

**21.** The method according to claim 20, wherein the redox mediator is ferrocinium, an osmium complex with bipyridine, benzophenone or ferricyanide.

**22.** The method according to any one of claims 1 to 21, wherein the sample comprises whole blood.

**23.** The method according to any one of claims 1 to 22, wherein the component comprises glucose.

**24.** The method according to any one of claims 1 to 23, wherein the reagent comprises PQQ dependent glucose dehydrogenase, NAD dependent glucose dehydrogenase, glucose oxidase, lactate dehydrogenase, or alcohol dehydrogenase.

**25.** The method according to any one of claims 1 to 24, wherein the potential is between +50 mV and +500 mV and is preferably about + 300 mV.

**26.** The method of claim 1, wherein the liquid sample is whole blood, the component of the liquid sample is glucose, the reagent (4) is PQQ dependent glucose dehydrogenase and the electroactive species is ferrocyanide, further comprising:

   providing a redox mediator comprising ferricyanide contained within the electrochemical cell; and
   placing the whole blood sample in the electrochemical cell such that the sample is in contact with the redox mediator,

wherein the step of reacting the component with the reagent to produce the electroactive species comprises reacting the glucose with the PQQ dependent glucose dehydrogenase to produce reduced PQQ dependent glucose dehydrogenase, the reduced PQQ dependent glucose dehydrogenase in tum reacting with the ferricyanide redox mediator to form ferrocyanide,
and wherein the measurement of current produced by the electrochemical reaction of ferrocyanide at the working electrode (2) is indicative of the rate of the chemical reaction between glucose and PQQ dependent glucose dehydrogenase.

**Patentansprüche**

1. Verfahren zum Messen einer Geschwindigkeit einer chemischen Reaktion zwischen einer Komponente einer flüssigen Probe und einem Reagens, wobei die Reaktion eine elektroaktive Spezies erzeugt, welches umfaßt:

> Bereitstellen einer elektrochemischen Zelle mit einer Arbeitselektrode (2), einer Gegenelektrode (3) und wenigstens einer Wand (1);
> im wesentlichen Immobilisieren des Reagens (4) in der elektrochemischen Zelle an einer Stelle (5) in einem minimalen Abstand von der Arbeitselektrode (2), wobei der Abstand so ist, daß eine Überführung der elektroaktiven Spezies von der Stelle (5) zur Arbeitselektrode (2) diffusionsgesteuert ist;
> Anordnen der flüssigen Probe in der elektrochemischen Zelle, so daß die flüssige Probe in Kontakt mit dem Reagens (4), der Arbeitselektrode (2) und der Gegenelektrode (3) ist;
> Umsetzen der Komponente mit dem Reagens (4), um die elektroaktive Spezies zu erzeugen;
> Beaufschlagen eines Potentials zwischen der Arbeitselektrode (2) und der Gegenelektrode (3), wobei das Potential ausreichend ist, um die elektroaktive Spezies an der Arbeitselektrode (2) elektrochemisch umzusetzen; und
> Messen des durch die chemische Reaktion an der Arbeitselektrode (2) erzeugten Stroms, um ein Maß der Geschwindigkeit der chemischen Reaktion zu erhalten,

> wobei die Geschwindigkeit der chemischen Reaktion gemäß der Gleichung dC/Dt = (L/FAD)di/dt bestimmt wird, wobei i der Strom ist, F die Faraday-Konstante ist, A die Elektrodenfläche ist, D der Diffusionskoeffizient der elektroaktiven Spezies in der Probe ist, C die Konzentration der elektroaktiven Spezies an der Stelle ist und L der Abstand zwischen der Stelle und der Elektrode ist.

2. Verfahren nach Anspruch 1, wobei die Arbeitselektrode (2) und die Gegenelektrode (3) ausreichend beabstandet sind, so daß ein Produkt einer elektrochemischen Reaktion, die an der Gegenelektrode (3) stattfindet, nicht die Arbeitselektrode (2) erreicht, während der Strom gemessen wird.

3. Verfahren nach Anspruch 2, wobei die Arbeitselektrode (2) und die Gegenelektrode (3) mit einem Abstand von größer als etwa 500 μm, bevorzugt zwischen 500 μm und 5 mm, bevorzugter zwischen 1 mm und 2 mm, beabstandet sind.

4. Verfahren nach Anspruch 2 oder Anspruch 3, wobei die Arbeitselektrode (2) und die Gegenelektrode (3) auf der gleichen Ebene untergebracht sind.

5. Verfahren nach einem der Ansprüche 1 bis 4, wobei die Stelle (5) und die Arbeitselektrode (2) durch einen minimalen Abstand im Bereich von 10 μm bis 5 mm, bevorzugt von 50 μm bis 500 μm, bevorzugter von 100 μm bis 200 μm, getrennt sind.

6. Verfahren nach einem der Ansprüche 1 bis 5, wobei die Gegenelektrode (3) fähig ist zur Funktion als eine kombinierte Gegen-Bezugs-Elektrode.

7. Verfahren nach einem der Ansprüche 1 bis 5, wobei die elektrochemische Zelle ferner eine Bezugselektrode umfaßt.

8. Verfahren nach einem der Ansprüche 1 bis 7, wobei die Arbeitselektrode (2) als eine Anode fungiert.

9. Verfahren nach Anspruch 8, wobei die Arbeitselektrode (2) Platin, Palladium, Kohlenstoff, Kohlenstoff in Kombination mit einem oder mehreren inerten Bindemitteln, Iridium, Indiumoxid, Zinnoxid, Indium in Kombination mit Zinnoxid oder eine Mischung derselben umfaßt.

10. Verfahren nach einem der Ansprüche 1 bis 7, wobei die Arbeitselektrode (2) als eine Kathode fungiert.

11. Verfahren nach einem der Ansprüche 1 bis 10, wobei die Gegenelektrode (3) Silber beschichtet mit einem im wesentlichen unlöslichen Silbersalz umfaßt.

12. Verfahren nach Anspruch 11, wobei das Silbersalz 5ilbercltlorid, Silberbromid, Silberiodid, Silbetferrocyaeid oder Silberferncyanid ist.

13. Verfahren nach einem der Ansprüche 1 bis 12, wobei die Stelle (5) an der Gegenelektrode (3) untergebracht ist.

14. Verfahren nach einem der Ansprüche 1 bis 13, wobei die Stelle (5) an der Wand untergebracht ist.

15. Verfahren nach einem der Ansprüche 1 bis 14, wobei die Stelle (5) und die Arbeitselektrode (2) auf der gleichen Ebene untergebracht sind.

16. Verfahren nach einem der Ansprüche 1 bis 14, wobei die Stelle (5) in einer Ebene zugewandt und im wesentlichen parallel zur Arbeitselektrode (2) untergebracht ist.

17. Verfahren nach einem der Ansprüche 1 bis 16, wobei das Reagens (4) innerhalb einer polymeren Matrix angefügt an einer Oberfläche in der elektrochemischen Zelle enthalten ist.

**18.** Verfahren nach einem der Ansprüche 1 bis 16, wobei das Reagens (4) chemisch oder physikalisch an einer Oberfläche in der elektrochemischen Zelle angebunden ist.

**19.** Verfahren nach einem der Ansprüche 1 bis 16, wobei das Reagens (4) auf einer Oberfläche in der elektrochemischen Zelle getrocknet wird, das Reagens (4) eine ausreichend geringe Mobilität in der flüssigen probe zeigt, so daß das Reagens (4) im wesentlichen nicht migriert, während der Strom gemessen wird.

**20.** Verfahren nach einem der Ansprüche 1 bis 19, wobei das Reagens (4) ferner einen Redoxmediator umfaßt.

**21.** Verfahren nach Anspruch 20, wobei der Redoxmediator Ferrocinium, ein Osmiumkomplex mit Bipyridin, Benzophenon oder Ferricyanid ist.

**22.** Verfahren nach einem der Ansprüche 1 bis 21, wobei die Probe Vollblut umfaßt.

**23.** Verfahren nach einem der Ansprüche 1 bis 22, wobei die Komponente Glucose umfaßt.

**24.** Verfahren nach einem der Ansprüche 1 bis 23, wobei das Reagens PQQ.abhängige Glucosedehydrogenase, NAD-abhättgige Glucosedehydrogenase, Glucoseoxidase, Lactatdehydrogenase oder Alkoholdehydrogenase umfaßt.

**25.** Verfahren nach einem der Ansprüche 1 bis 24, wobei das Potential zwischen +50 mV und +500 mV und bevorzugt etwa+300 mV ist.

**26.** Verfahren nach Anspruch 1, wobei die flüssige Probe Vollblut ist, die Komponente der flüssigen Probe Glucose ist, das Reagens (4) PQQ-abhängige Glucosedehyärogenase ist und die elektroaktive Spezies Ferrocyanid ist, weiter umfassend:

Bereitstellen eines Redoxmediators umfassend Ferricyanid enthaltend innerhalb der elektrochemischen Zelle; und
Anordnen der Vollblutprobe in der elektrochemischen Zelle, so daß die Probe in Kontakt mit dem Redoxmediator ist,

wobei der Schritt des Umsetzens der Komponente mit dem Reagens, um die elektroaktive Spezies zu erzeugen, ein Umsetzen der Glucose mit der PQQabhängigen Glucosedehydrogenase umfaßt, um reduzierte PQQ-abhängige Glucosedehydrogenase zu erzeugen, wobei die reduzierte PQQ-abhängige Glucosedehydrogenase wiederum mit dem Ferricyanid-Redoxmediator reagiert, um Ferrocyanid zu bilden,

und wobei die Messung des durch die elektrochemische Reaktion von Ferrocyanid an der Arbeitselektrode (2) erzeugten Stroms anzeigend ist für die Geschwindigkeit der chemischen Reaktion zwischen Glucose und PQQ-abhängiger Glucosedehydrogenase.

**Revendications**

**1.** Procédé de mesure d'une vitesse d'une réaction chimique entre un composant d'un échantillon liquide et un réactif, la réaction produisant une espèce électroactive, comprenant les étapes consistant à :

fournir une cellule électrochimique ayant une électrode à étudier (2), une contre-électrode (3) et au moins une paroi (1);
immobiliser sensiblement le réactif (4) dans la cellule électrochimique au niveau d'un site (5) à une distance minimale de l'électrode à étudier (2), dans lequel la distance est telle qu'un transfert de l'espèce électroactive depuis le site (5) vers l'électrode à étudier (2) est contrôlé par diffusion ;
placer l'échantillon liquide dans la cellule électrochimique de telle sorte que l'échantillon liquide soit en contact avec le réactif (4), l'électrode à étudier (2) et la contre-électrode (3) ;
faire réagir le composant avec le réactif (4) pour produire l'espèce électroactive ;
appliquer un potentiel entre l'électrode à étudier (2) et la contre-électrode (3), dans lequel le potentiel est suffisant pour faire réagir électrochimiquement l'espèce électroactive au niveau de l'électrode à étudier (2) ; et
mesurer l'intensité produite par la réaction électrochimique au niveau de l'électrode à étudier (2) pour obtenir une mesure de la vitesse de la réaction chimique,

dans lequel la vitesse de la réaction chimique est déterminée conformément à l'équation $dC/Dt = (L/FAD)di/dt$, où i est l'intensité, F est la constante de Faraday, A est la surface de l'électrode, D est le coefficient de diffusion de l'espèce électroactive dans l'échantillon, C est la concentration de l'espèce électroactive au niveau du site et L est la distance entre le site et l'électrode.

**2.** Procédé selon la revendication 1, dans lequel l'électrode à étudier (2) et la contre-électrode (3) sont suffisamment espacées pour qu'un produit d'une réaction électrochimique se produisant au niveau de la contre-électrode (3) n'atteigne pas l'électrode à étudier (2) alors que l'on mesure l'intensité.

**3.** Procédé selon la revendication 2, dans lequel l'électrode à étudier (2) et la contre-électrode (3) sont séparées d'une distance supérieure à environ 500 microns, de préférence d'une distance comprise entre 500 microns et 5 mm, de préférence entre 1 mm et 2 mm.

4.  Procédé selon la revendication 2 ou 3, dans lequel l'électrode à étudier (2) et la contre-électrode (3) sont situées sur le même plan.

5.  Procédé selon l'une quelconque des revendications 1 à 4, dans lequel le site (5) et l'électrode à étudier (2) sont séparés d'une distance minimale comprise entre 10 microns et 5 millimètres, de préférence entre 50 microns et 500 microns, de préférence entre 100 microns et 200 microns.

6.  Procédé selon l'une quelconque des revendications 1 à 5, dans lequel la contre-électrode (3) est capable de fonctionner comme une électrode combinée (contre-électrode et électrode de référence).

7.  Procédé selon l'une quelconque des revendications 1 à 5, dans lequel la cellule électrochimique comprend en outre une électrode de référence.

8.  Procédé selon l'une quelconque des revendications 1 à 7, dans lequel l'électrode à étudier (2) fonctionne comme une anode.

9.  Procédé selon la revendication 8, dans lequel l'électrode à étudier (2) comprend du platine, du palladium, du carbone, du carbone combiné avec un ou plusieurs liants inertes, de l'iridium, de l'oxyde d'indium, de l'oxyde d'étain, de l'indium combiné avec de l'oxyde d'étain ou un mélange de ceux-ci.

10. Procédé selon l'une quelconque des revendications 1 à 7, dans lequel l'électrode à étudier (2) fonctionne comme une cathode.

11. Procédé selon l'une quelconque des revendications 1 à 10, dans lequel la contre-électrode (3) comprend de l'argent recouvert avec un sel d'argent sensiblement insoluble.

12. Procédé selon la revendications 11, dans lequel le sel d'argent est le chlorure d'argent, le bromure d'argent, l'iodure d'argent, le ferrocyanure d'argent ou le ferricyanure d'argent.

13. Procédé selon l'une quelconque des revendications 1 à 12, dans lequel le site (5) est situé sur la contre-électrode (3).

14. Procédé selon l'une quelconque des revendications 1 à 13, dans lequel le site (5) est situé sur la paroi.

15. Procédé selon l'une quelconque des revendications 1 à 14, dans lequel le site (5) et l'électrode à étudier (2) sont situés sur le même plan.

16. Procédé selon l'une quelconque des revendications 1 à 14, dans lequel le site (5) est situé dans un plan face et sensiblement parallèle à l'électrode à étudier (2).

17. Procédé selon l'une quelconque des revendications 1 à 16, dans lequel le réactif (4) est contenu à l'intérieur d'une matrice polymère fixée à une surface dans la cellule électrochimique.

18. Procédé selon l'une quelconque des revendications 1 à 16, dans lequel le réactif (4) est chimiquement maintenu ou physiquement maintenu à une surface dans la cellule électrochimique.

19. Procédé selon l'une quelconque des revendications 1 à 16, dans lequel le réactif (4) est séché sur une surface dans la cellule électrochimique, le réactif (4) présentant une mobilité suffisamment faible dans l'échantillon liquide pour que le réactif (4) ne migre sensiblement pas alors que l'on mesure l'intensité.

20. Procédé selon l'une quelconque des revendications 1 à 19, dans lequel le réactif (4) comprend en outre un médiateur redox.

21. Procédé selon la revendication 20, dans lequel le médiateur redox est le ferrocinium, un complexe d'osmium avec de la bipyridine, du benzophénone ou du ferricyanure.

22. Procédé selon l'une quelconque des revendications 1 à 21, dans lequel l'échantillon comprend du sang entier.

23. Procédé selon l'une quelconque des revendications 1 à 22, dans lequel le composant comprend du glucose.

24. Procédé selon l'une quelconque des revendications 1 à 23, dans lequel le réactif comprend une glucose déshydrogénase PQQ, une glucose déshydrogénase à NAD, une glucose oxydase, une lactate déshydrogénase ou une alcool déshydrogénase.

25. Procédé selon l'une quelconque des revendications 1 à 24, dans lequel le potentiel est compris entre + 50 mV et + 500 mV et vaut de préférence environ + 300 mV.

26. Procédé selon la revendication 1, dans lequel l'échantillon liquide est du sang entier, le composant de l'échantillon liquide est le glucose, le réactif (4) est la glucose déshydrogénase PQQ et l'espèce électroactive est le ferrocyanure, comprenant en outre les étapes consistant à :

fournir un médiateur redox comprenant du ferrocyanure contenu à l'intérieur de la cellule électrochimique ; et
placer l'échantillon de sang entier dans la cellule électrochimique de telle sorte que l'échantillon soit en contact avec le médiateur redox,

dans lequel l'étape consistant à faire réagir le composant avec le réactif pour produire l'espèce électroactive comprend la réaction du glucose avec la glucose déshydrogénase PQQ pour produire une glucose déshydrogénase PQQ réduite, la glucose déshydrogénase PQQ réduite réagissant à son tour avec le médiateur redox de ferricyanure pour former du ferrocyanure,
et dans lequel la mesure de l'intensité produite par la réaction électrochimique du ferrocyanure au niveau de l'électrode à étudier (2) est indicatrice de la vitesse de la réaction chimique entre le glucose et la glucose déshydrogénase PQQ.

*Fig. 1*

*Fig. 2*

*Fig. 3*